(19) Europäisches Patentamt
European Patent Office
Office européen des brevets

(11) Publication number: **0 074 724**
**A2**

(12) # EUROPEAN PATENT APPLICATION

(21) Application number: 82304380.7

(22) Date of filing: 19.08.82

(51) Int. Cl.³: C 10 M 1/32
C 10 L 1/22, C 07 D 233/12

(30) Priority: 03.09.81 US 298970

(43) Date of publication of application:
23.03.83 Bulletin 83/12

(84) Designated Contracting States:
BE DE FR GB IT NL SE

(71) Applicant: The Lubrizol Corporation
29400 Lakeland Boulevard
Wickliffe, Ohio 44092(US)

(72) Inventor: Koch, Frederick William The Lubrizol Corporation
Patent Department 29400 Lakeland Boulevard
Wickliffe Ohio 44092(US)

(72) Inventor: Floyd, Robert L. The Lubrizol Corporation
Patent Department 29400 Lakeland Boulevard
Wickliffe Ohio 44092(US)

(74) Representative: Thomas, Roger Tamlyn et al,
D. Young & Co. 10 Staple Inn
London WC1V 7RD(GB)

(54) **Acylated imidazolines and fuel and lubricant compositions thereof.**

(57) Novel additive compositions are provided comprising the reaction product of a mixture of reactants comprising (A) an unsubstituted or aliphatic hydrocarbon based substituted succinic acylating agent and (B) an imidazoline. Fuels and lubricants containing said novel additive compositions are also described.

EP 0 074 724 A2

TITLE: ACYLATED IMIDAZOLINES AND FUEL AND LUBRICANT COMPOSITIONS THEREOF

Field of the Invention

This invention relates to novel additive compositions for use in lubricants and normally liquid fuels. More particularly, this invention relates to novel additive compositions derived from the interreaction of an unsubstituted succinic acylating agent or an aliphatic hydrocarbon based substituted acylating agent and an imidazoline, to concentrates thereof and to lubricants and normally liquid fuels containing said additive compositions.

Description of the Prior Art

It is well-known that lubricants and normally liquid fuels can cause corrosion of various materials with which they may be brought into contact. For example, it is well-known that normally liquid fuels tend to accumulate considerable quantities of water during storage and when subsequently brought into contact with metal surfaces in their functional environment cause deterioration of such surfaces as a result of the formation of rust and corrosion.

It is also known that water is a common contaminant in crankcase lubricants for engines. It may result from the oxidation of the lubricating oil or come from the combustion chamber as a blow-by product of the burning fuel. The presence of this water, in addition to causing rust and corrosion, also seems to promote the deposition of a mayonnaise-like sludge. This type of sludge is even more objectionable in that it clings tenaciously to metal surfaces and is not removed through filtering.

To overcome the corrosive nature of lubricants and normally liquid fuels and to improve the dispersant properties of lubricants, it is common practice to add to such lubricants and fuels minor proportions of anti-corrosion and dispersant materials. Many materials have been developed for these purposes including, for example, imidazoline salts of mono- and dicresyl phosphates (U.S. Pat. No. 3,736,110), mixtures of alkyl or alkenyl succinic acids and N-aminoalkyl or N-hydroxyalkyl substituted imidazoline salts of alkyl or alkenyl succinic acids (U.S. Pat. No. 3,282,836), metal alkyl or alkoxy metal alkyl, ester tetrapropenyl succinates (U.S. Pat. No. 3,485,858), polymerized olefin substituted succinic acid esters (U.S. Pat. No. 3,381,022), and reaction products of partially esterified succinic acid compounds and hydroxyalkylated derivatives of a partially acylated polyamines (U.S. Pat. No. 3,240,575), to name but a few and the search continues for others.

Summary of the Invention

In the broadest sense, the additive compositions of the present invention are reaction products prepared by the interreaction of a mixture of reactants comprising (A) at least one unsubstituted or aliphatic hydrocarbon based substituted succinic acylating agent and (B) at least one imidazoline of the formula

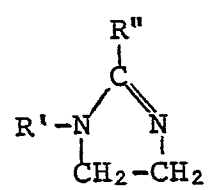

where R' is selected from the group consisting of hydrogen and hydrocarbon based radicals and R" is an aliphatic or alicyclic hydrocarbon based radical. In a more preferred embodiment the additive compositions of the present invention are reaction products prepared by the interreaction of a mixture of reactants comprising (A) an aliphatic hydrocarbon based substituted succinic acylating agent wherein the aliphatic hydrocarbon based substituent is a straight-chain or branched-chain alkyl or alkenyl radical containing from about 4 to about 100 carbon atoms and (B) an imidazoline of the formula above wherein R' is a straight-chain or branched-chain aliphatic hydrocarbon based radical substituted with -OH or $-NH_2$ groups and containing from about 1 to about 6 carbon atoms and R" is an aliphatic or alicyclic hydrocarbon based radical containing from about 10 to about 40 carbon atoms. In yet a more preferred embodiment the additive compositions of this invention are products prepared by the interreaction of a mixture of reactants comprising (A) an alkenyl substituted succinic acylating agent wherein the alkenyl substituent contains from 8 to 30 carbon atoms and (B) an imidazoline of the formula above wherein R' is a straight-chain or branched-chain hydroxyalkyl radical containing from about 1 to about 6 carbon atoms and R" is a straight-chain or branched-chain aliphatic hydrocarbon based radical containing from about 10 to about 20 carbon atoms. In yet a more preferred embodiment the additive compositions of this invention are reaction products prepared by the interreaction of a mixture of reactants comprising (A) an alkenyl substituted succinic acylating agent wherein the alkenyl substituent contains from about 9 to about 15 carbon atoms and (B) an imidazoline of the above formula wherein R' is a straight-chain or branched-chain hydroxyalkyl radical containing from about 2 to about 4 carbon atoms and R" is a straight-chain or branched-chain alkenyl radical containing from about 10 to about 20 carbon atoms. In a most preferred embodiment the additive compositions of the present invention are products prepared by the interreaction of a mixture of reactants comprising (A) alkenyl substituted succinic anhydride wherein the alkenyl substituent contains about 12 carbon atoms and (B) an imidazoline of the formula above where R' is a straight-chain hydroxyalkyl radical containing 2 carbon atoms and

R" is a straight-chain alkenyl radical containing about 17 carbon atoms.

Further embodiments of this invention include lubricants and normally liquid fuel compositions comprising a major proportion of the lubricant or normally liquid fuel and a minor proportion of the additive compositions defined and described herein and to concentrates of said additive compositions.

As used herein, the term "hydrocarbon-based radical" denotes a radical having a carbon atom directly attached to the remainder of the molecule and having predominantly hydrocarbon character within the context of this invention. Such radicals include the following:

(1) Hydrocarbon radicals; that is, aliphatic, (e.g., alkyl or alkenyl), alicyclic (e.g., cycloalkyl or cycloalkenyl), aromatic, aliphatic- and alicyclic-substituted aromatic, aromatic-substituted aliphatic and alicyclic radicals, and the like, as well as cyclic radicals wherein the ring is completed through another portion of the molecule (that is, any two indicated substituents may together form an alicyclic radical). Such radicals are known to those skilled in the art; examples include methyl, ethyl, propyl, butyl, octyl, decyl, dodecyl, cyclohexyl, phenyl, tolyl, benzyl and the like.

(2) Substituted hydrocarbon radicals; that is, radicals containing non-hydrocarbon substituents which, in the context of this invention, do not alter the predominantly hydrocarbon character of the radical. Those skilled in the art will be aware of suitable substituents (hydroxy, alkoxy, nitro, carbalkoxy).

(3) Hetero radicals; that is, radicals which, while predominantly hydrocarbon in character within the context of this invention, contain atoms other than carbon present in a chain or ring otherwise composed of carbon atoms. Suitable hetero atoms will be apparent to those skilled in the art and include, for example, nitrogen, oxygen and sulfur.

Detailed Description of the Preferred Embodiments

The additive compositions of the present invention are complex products obtained by the interreaction of at least two separate components. Component (A) is an unsubstituted or aliphatic hydrocarbon based substituted succinic acylating agent and component (B) an imidazoline.

The unsubstituted and aliphatic hydrocarbon based substituted succinic acylating agents, component (A), useful in preparing the additive compositions of this invention are the unsubstituted and aliphatic hydrocarbon based substituted succinic acids and anhydrides thereof having the following structural formulas

Anhydride

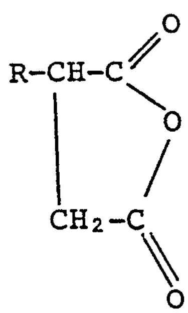

and

Acid

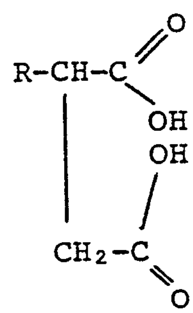

where R is hydrogen or an aliphatic hydrocarbon based radical containing from about 4 to about 100 carbon atoms, preferably from about 8 to about 30 carbon atoms and most preferably from about 9 to about 15 carbon atoms. Although the source of the aliphatic hydrocarbon based substituent is not a critical aspect of the invention, this substituent will generally be derived from various olefinic monomers such as ethylene, propylene, butylene, hexene, octene, decene, etc., including the oligomers, prepolymers and low molecular weight polymers formed from the foregoing monomers. Thus dimers, trimers and/or tetramers of propylene and butylene can be used.

The aliphatic hydrocarbon based substituted succinic acylating agents, component (A), are the preferred acylating agents for use in preparing the additive compositions of this invention. They are well-known and can be prepared by known procedures. One particularly useful procedure is to react a monoolefin monomer or oligomer as

described above with maleic anhydride at 100°C to 200°C with or without a catalyst to form the corresponding substituted succinic anhydride. The monoolefin can be unsaturated in the alpha position such as in 1-butene, 1-hexene, 1-octene and 1-decene, or in a medial position such as in 2-butene, 3-pentene, 4-octene and the like. The same is true of the oligomers. The monoolefin also can be replaced by an alkyl halide which is capable of being subsituted onto the unsaturated anhydride or the equivalent free acid thereof.

The aliphatic hydrocarbon based substituent can be saturated or unsaturated, straight-chain or branched-chain and may contain polar groups provided, however, that such groups are not present in significantly large proportions as to alter the hydrocarbon character of the substituent. Polar groups are typified by halo, carbonyl, nitro and similar groups.

The imidazolines, component (B), that can be employed to prepare the additive compositions of this invention are represented by the formula

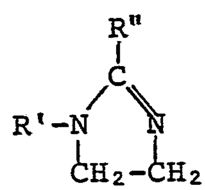

where R' is selected from the group consisting of hydrogen and hydrocarbon based radicals and R" is an aliphatic or alicyclic hydrocarbon based radical. In a preferred embodiment R' is a straight-chain or branched-chain aliphatic hydrocarbon based radical substituted with -OH or $-NH_2$ groups, preferably -OH groups, and containing from 1 to 6 and preferably 2 to 4 carbon atoms and R" will contain from about 10 to about 20 carbon atoms. The most preferred imidazoline is the one where R' is 2-hydroxyethyl radical and R" is 2-heptadecenyl radical. Non-limiting examples of other useful imidazolines include 2-heptadecenyl-1-(4-hydroxybutylimidazoline); 2-dodecyl-1-(5-hydroxypentylimidazoline); 2-(3-cyclohexylpropyl)-1-(2-hydroxyethylimidazoline); 2-dodecylcyclohexyl-1-(2-hydroxyethylimidazoline); 2-

heptadecenyl-1-butylimidazoline; 2-tridecyl-1-(2-aminoethylimidazoline); 2-heptadecenyl-1-(2-aminoethylimidazoline) and the like.

The above imidazolines can be prepared by methods such as those disclosed in U.S. Pat. No. 2,267,965 and U.S. Pat. No. 2,214,152. Generally the imidazolines of the above formula are readily formed by reacting an aliphatic or alicyclic carboxylic acid with an appropriate unsubstituted or hydrocarbon based radical substituted ethylene diamine. The reaction involves condensation of the acid with the diamine, at a temperature ranging from about 110°C to about 350°C, with the elimination of 2 moles of water. The reaction is represented by the following equation

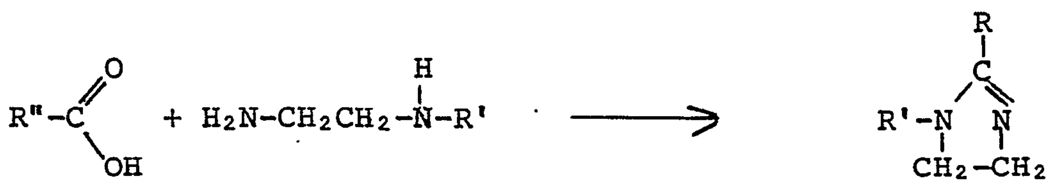

where R' and R" have the same meaning as in the formula above.

The aliphatic or alicyclic carboxylic acid may be saturated or unsaturated and may contain substituents such as halo, ether, sulfide, keto, hydroxyl, etc., as well as phenyl, tolyl, xylyl, chlorophenyl, hydroxyphenyl, naphthyl, methyl-naphthyl, etc. Representative, but non-limiting examples of acids useful for the preparation of the imidazolines adapted for the purposes of this invention include undecanoic, myristic, palmitic, stearic, oleic, linoleic, linolenic, ricinoleic, behenolic, stearolic phenylstearic, xylylstearic, chlorostearic, hydroxyphenylstearic, arachidic, tricosanoic, triacontanoic and dimer acids and the like. Mixtures of any of the foregoing acids are likewise useful.

The additive compositions of the present invention may be readily prepared by heating mixtures of the unsubstituted or aliphatic hydrocarbon based substituted succinic acylating agent, component (A), with the imidazoline, component (B), at elevated temperatures ranging from about

30°C. to about 250°C. A more preferred range of reaction temperatures is from about 100°C. to about 200°C. particularly where it is preferred that the desired additive composition be of the ester/salt, ester/amide, amide/salt or imide type. Below about 80°C. the additive compositions are believed to be predominately salts or mixed salts/esters. Above about 80°C. the ester/salt, ester/amide, amide/salt and imide types of structures are believed to predominate.

The molar ratio of component (A) to component (B) in the mixture of reactants can range from about 1.0:0.5 to about 1.0:3.0. The preferred molar ratio ranges from about 1.0:1.0 to about 1.0:2.0.

The additive compositions of this invention can be prepared either in the absence or presence of a solvent. Preferably the additive compositions of this invention are prepared in the presence of a solvent. Solvents suitable for use in the reaction may be hydrocarbon or polar solvents such as, for example, benzene, naphtha, toluene, xylene, n-hexane, dioxane, chlorobenzene, kerosene, gasoline or a fuel oil and oil of lubricating viscosity.

The exact chemical constitution of the additive compositions of this invention are not known with certainty although from the nature of the starting reactants used and the reaction conditions employed salts, mixed salts/esters, ester/amide, amide/salt and imide type compositions and mixtures thereof are believed to be obtained.

Following are examples illustrating preparations of the additive compositions of this invention. All parts are by weight unless otherwise specified.

## Example 1

Two hundred sixty-six parts tetrapropenyl substituted succinic anhydride (prepared by reacting maleic anhydride with a polypropylene tetramer) is charged to a reactor equipped with a stirrer, thermometer, reflux condenser and addition funnel. Seven hundred and eight parts of 2-heptadecenyl-1-(2-hydroxyethylimidazoline) is then added dropwise to the anhydride while the temperature rises

exothermically from room temperature to 62°C. Upon completion of the imidazoline addition, two hundred forty-three parts diluent oil is added and the solution is stirred for 1.5 hours without additional heating and filtered. The product obtained has neutralization numbers of 47 acid to phenolphthalein and 69 basic to bromophenol blue.

Example 2

One thousand sixty-four parts polypropenyl succinic anhydride were charged to a reactor equipped as described in Example 1. Then 2,832 parts of 2-heptadecenyl-1-(2-hydroxyethylimidazoline) are added dropwise with vigorous stirring over a period of 2.5 hours. During addition, the temperature is kept between 68°-75°C. The reaction mixture is then heated an additional one hour at 80°C. The product obtained has neutralization numbers of 55 acid to phenolphthalein and 108 basic to bromophenol blue. This product was then heated an additional 3 hours at 140°-150°C. to give a final product having neutralization numbers of 815 acid to phenolphthalein and 56 basic to bromophenol blue.

Example 3

Four hundred thirty-four parts polyisobutenyl (molecular weight about 300) succinic anhydride (prepared by reacting maleic anhydride with chlorinated polyisobutene), 350 parts 2-heptadecenyl-1-(2-hydroxyethylimidazoline) and 525 parts diluent oil are charged to a reactor equipped with a stirrer, condenser, thermometer, nitrogen inlet tube and a Dean-Stark trap. The materials are heated to 160°C. with a nitrogen purge over six hours. Water is collected in the Dean-Stark trap. The materials are stripped to 160°C. at 15 torr. The residue is filtered and collected. The product obtained contains 2.40% nitrogen.

Example 4

One thousand five hundred thirty-five parts polyisobutenyl (molecular weight about 900) succinic anhydride (prepared by reacting maleic anhydride with chlorinated polyisobutene) is charged to a reactor equipped with a

stirrer, thermometer and condenser. 2-heptadecenyl-1-(2-hydroxyethylimidazoline) is added dropwise with vigorous stirring over a period of one hour at a temperature of 60-90°C. The materials are then heated for an additional five hours at a temperature between 100-130°C. The filtered product obtained has neutralization numbers of 17 acid to phenolphthalein and 9 basic to bromophenol blue.

Example 5

Five hundred sixty parts polyisobutenyl (molecular weight about 900) succinic anhydride, 350 parts of 2-heptadecenyl-1-(2-hydroxyethylimidazoline) and about 100 parts xylene are charged to a reactor equipped with a stirrer, thermometer and Dean-Stark trap and heated to 172°C. and held at this temperature for three hours. No water is evolved. A total of 1.5 milliliters $H_2SO_4$ is then added and the mixture is refluxed for six additional hours. (About 4 milliliters $H_2O$ is obtained). Additional xylene is added for a total of 386 parts and then the solution is filtered. The product obtained has neutralization numbers of 10.7 basic to bromophenol blue and 2.2 acid to phenolphthalein.

Example 6

One thousand eighty parts polyisobutenyl (molecular weight about 900) succinic anhydride and one thousand one hundred seventy parts diluent oil is charged to a reactor equipped as described in Example 5. The oil solution is heated to 100°C. and 350 parts of 2-heptadecenyl-1-(2-hydroxyethylimidazoline) are added over one hour. The reaction mixture is stripped at a temperature of 160°C. under a nitrogen purge and filtered. The product obtained contains 1.14% nitrogen and has neutralization numbers of 3.0 basic to bromophenol blue and 0.8 acid to phenolphthalein.

Example 7

One thousand five hundred fifty-two parts of a solution containing 40% diluent oil of polyisobutenyl (molecular weight about 900) succinic anhydride is charged to a reactor equipped with a stirrer, thermometer and condenser. Two hundred sixty-nine parts of 2-heptadecenyl-

1-(2-hydroxyethylimidazoline) are added with vigorous stirring over 0.75 hours at 45-60°C. The product is heated for an additional two hours between 50-60°C. The product obtained has neutralization numbers of 47 acid to phenolphthalein and 21 basic to bromophenol blue.

Example 8

The procedure of Example 7 is followed except five hundred thirty-eight parts 2-heptadecenyl-1-(2-hydroxyethylimidazoline) is used. The product obtained has neutralization numbers of 42 acid to phenolphthalein and 35 basic to bromophenol blue.

Example 9

Nine hundred fifty-two parts polypropenyl succinic acid (prepared by reacting polypropenyl succinic anhydride with water) is charged to a reactor equipped with a stirrer, thermometer and condenser. Seven hundred eighteen parts of 2-heptadecenyl-1-(2-hydroxyethylimidazoline) is added dropwise over one hour between 55-75°C. The material is heated for an additional two hours between 55-75°C. The product obtained has neutralization numbers of 136 acid to phenolphthalein and 68 basic to bromophenol blue.

Example 10

Four hundred fifty parts polypropenyl succinic acid is charged to a reactor equipped with a stirrer, thermometer and condenser. Seven hundred eight parts of 2-heptadecenyl-1-(2-hydroxyethylimidazoline) is then added to the polypropenyl succinic acid while the temperature increases exothermically to 45°C. Four hundred fifteen parts of diluent oil is added and stirring is continued for an additional 0.75 hours. The product obtained has neutralization numbers of 67 acid to phenolphthalein and 64 basic to bromophenol blue.

Another embodiment of this invention is fuel compositions containing a minor proportion of the above described additives. The major proportion of the fuel composition comprises a normally liquid fuel, usually a hydrocarbonaceous petroleum distillate fuel such as motor

gasoline as defined by ASTM Specification D439 and diesel fuel or fuel oil as defined by ASTM Specification D396. Normally liquid fuel compositions comprising non-hydrocarbonaceous materials such as alcohols, ethers, organonitro compounds and the like (e.g., methanol, ethanol, hydrated ethanol, diethyl ether, methyl ethyl ether, nitromethane) are also within the scope of this invention as are liquid fuels derived from vegetable or mineral sources such as corn, alfalfa, shale and coal. Normally liquid fuels which are mixtures of one or more hydrocarbonaceous fuels and one or more non-hydrocarbonaceous materials are also contemplated. Examples of such mixtures are combinations of gasoline and ethanol, both hydrated and substantially anhydrous ethanols, and of diesel fuel and ether. Particularly preferred is gasoline, that is, a mixture of hydrocarbons having an ASTM distillation range from about 60°C. at the 10% distillation point to about 205°C. at the 90% distillation point, both hydrated and substantially anhydrous ethanols and mixtures of gasoline and methanol or said ethanols.

Generally, these fuel compositions contain an amount of the additive compositions of this invention sufficient to provide it with anti-corrosion properties; usually this amount is about 0.5 to about 2500 parts by weight, preferably 10 to 1000 parts, of the composition of this invention per million parts of fuel.

The fuel compositions can contain, in addition to the composition of this invention, other additives which are well known to those of skill in the art. These include antiknock agents such as tetraalkyl lead compounds, methyl-t-butyl ether, mixtures of methyl alcohol and t-butyl alcohol, lead scavengers such as haloalkanes (e.g., ethylene dichloride and ethylene dibromide), deposit preventers or modifiers such as triaryl phosphates, dyes, octane improvers, antioxidants such as 2,6-di-tertiary-butyl-4-methylphenol, bacteriostatic agents, gum inhibitors, metal deactivators, demulsifiers, upper cylinder lubricants and

anti-icing agents.

In certain preferred fuel compositions the compositions of this invention are combined with an ashless dispersant in gasoline. Suitable ashless dispersants include esters of mono- or polyols and high molecular weight mono- or polycarboxylic acid acylating agents containing at least 30 carbon atoms in the acyl moiety. Such esters are well known to those skilled in the art. See, for example, French Patent 1,396,645, British Patents 981,850; 1,055,337 and 1,306,529; and U.S. Patents 3,255,108; 3,311,558; 3,331,776; 3,346,354; 3,522,179; 3,579,450; 3,542,680; 3,381,022; 3,639,242; 3,697,428 and 3,708,522. These patents are expressly incorporated herein by reference for their disclosure of suitable esters and methods for their preparation. Generally, the weight ratio of the composition of this invention to the aforesaid ashless dispersant is between about 0.1:1 and about 10:1, preferably between about 1:1 and about 10:1.

The compositions of this invention can be added directly to the fuel, or they can be diluted with a substantially inert, normally liquid organic diluent such as naphtha, benzene, toluene, xylene or a normally liquid fuel as described above, to form an additive concentrate. These concentrates which constitute yet another embodiment of the present invention generally contain from about 20% to about 90% by weight of the composition of this invention and may contain, in addition one or more other conventional additives known in the art or described hereinabove.

As previously indicated, the compositions of this invention are also also useful as additives for lubricants. They can be employed in a variety of lubricants based on diverse oils of lubricating viscosity, including natural and synthetic lubricating oils and mixtures thereof. These lubricants include crankcase lubricating oils for spark-ignited and compression-ignited internal combustion engines, including automobile and truck engines, two-cycle engines, aviation piston engines, marine and railroad diesel engines, and the like. The compositions can also be used in gas

engines, stationary power engines and turbines and the like. Automatic transmission fluids, transaxle lubricants, gear lubricants, metal-working lubricants, hydraulic fluids and other lubricating oil and grease compositions can also benefit from the incorporation therein of the compositions of this invention.

Natural oils include liquid petroleum oils and solvent-treated or acid-treated mineral lubricating oils of the paraffinic, naphthenic and mixed paraffinic-naphthenic types. Oils of lubricating viscosity derived from coal or shale are also useful base oils.

Synthetic lubricating oils include hydrocarbon oils and halo-substituted hydrocarbon oils such as polymerized and interpolymerized olefins [e.g., polybutylenes, polypropylenes, propylene-isobutylene copolymers, chlorinated polybutylenes, poly(1-hexenes), poly(1-octenes), poly(1-decenes)]; alkylbenzenes [e.g., dodecylbenzenes, tetradecylbenzenes, dinonylbenzenes, di(2-ethylhexyl)benzenes]; polyphenyls (e.g., biphenyls, terphenyls, alkylated polyphenyls); and alkylated diphenyl ethers and alkylated diphenyl sulfides and the derivatives, analogs and homologs thereof.

Alkylene oxide polymers and interpolymers and derivatives thereof where the terminal hydroxyl groups have been modified by esterification, etherification, etc., constitute another class of known synthetic lubricating oils. These are exemplified by polyoxyalkylene polymers prepared by polymerization of ethylene oxide or propylene oxide, the alkyl and aryl ethers of these polyoxyalkylene polymers (e.g., methyl-polyisopropylene glycol ether having an average molecular weight of 1000, diphenyl ether of polyethylene glycol having a molecular weight of 500-1000, diethyl ether of polypropylene glycol having a molecular weight of 1000-1500); and mono- and polycarboxylic esters thereof, for example, the acetic acid esters, mixed $C_3$-$C_8$ fatty acid esters and $C_{13}$ Oxo acid diester of tetraethylene glycol.

Another suitable class of synthetic lubricating oils comprises the esters of dicarboxylic acids (e.g., phthalic acid, succinic acid, alkyl succinic acids and alkenyl succinic acids, maleic acid, azelaic acid, suberic acid, sebacic acid, fumaric acid, adipic acid, linoleic acid dimer, malonic acid, alkyl malonic acids, alkenyl malonic acids) with a variety of alcohols (e.g., butyl alcohol, hexyl alcohol, dodecyl alcohol, 2-ethylhexyl alcohol, ethylene glycol, diethylene glycol monoether, propylene glycol). Specific examples of these esters include dibutyl adipate, di(2-ethylhexyl) sebacate, di-n-hexyl fumarate, dioctyl sebacate, diisooctyl azelate, diisodecyl azelate, dioctyl phthalate, didecyl phthalate, dieicosyl sebacate, the 2-ethylhexyl diester of linoleic acid dimer, and the complex ester formed by reacting one mole of sebacic acid with two moles of tetraethylene glycol and two moles of 2-ethylhexanoic acid.

Esters useful as synthetic oils also include those made from $C_5$ to $C_{12}$ monocarboxylic acids and polyols and polyol ethers such as neopentyl glycol, trimethylolpropane, pentaerythritol, dipentaerythritol and tripentaerythritol.

Silicon-based oils such as the polyalkyl-, polyaryl-, polyalkoxy-, or polyaryloxysiloxane oils and silicate oils comprise another useful class of synthetic lubricants; they include tetraethyl silicate, tetraisopropyl silicate, tetra-(2-ethylhexyl) silicate, tetra-(4-methyl-2-ethylhexyl) silicate, tetra-(p-tert-butylphenyl) silicate, hexa-(4-methyl-2-pentoxy)disiloxane, poly(methyl) siloxanes and poly(methylphenyl)siloxanes. Other synthetic lubricating oils include liquid esters of phosphorus-containing acids (e.g., tricresyl phosphate, trioctyl phosphate, diethyl ester of decylphosphonic acid) and polymeric tetrahydrofurans.

Unrefined, refined and rerefined oils can be used in the lubricants of the present invention. Unrefined oils are those obtained directly from a natural or synthetic source without further purification treatment. For example,

a shale oil obtained directly from retorting operations, a petroleum oil obtained directly from distillation or ester oil obtained directly from an esterification process and used without further treatment would be an unrefined oil. Refined oils are similar to the unrefined oils except they have been further treated in one or more purification steps to improve one or more properties. Many such purification techniques, such as distillation, solvent extraction, acid or base extraction, filtration and percolation are known to those skilled in the art. Rerefined oils are obtained by processes similar to those used to obtain refined oils applied to refined oils which have been already used in service. Such rerefined oils are also known as reclaimed or reprocessed oils and often are additionally processed by techniques for removal of spent additives and oil breakdown products.

Generally, the lubricants of the present invention contain an amount of the additive compositions of this invention sufficient to provide it with anti-corrosion and dispersant properties. Normally this amount will be about 0.05 percent to about 10.0 percent by weight and preferably from about 0.1 percent to about 5.0 percent by weight of the additive compositions of this invention.

Illustrative of the fuel compositions of this invention are gasolines, substantially anhydrous ethanol, hydrated ethanol and methanol and mixtures of gasolines and said alcohols containing 800 parts of the additive compositions of Examples 1-10 per million parts of said fuels. Illustrative of the lubricant compositions of this invention are crankcase lubricants containing 2.0 percent by weight and turbine oils containing about 0.1 percent by weight based on the weight of the lubricant or oil of the additive compositions of Examples 1-10 herein.

CLAIMS

1. An additive for use in fuels and lubricants comprising the reaction product of a mixture of reactants comprising (A) at least one unsubstituted or aliphatic hydrocarbon based substituted succinic acylating agent and (B) at least one imidazoline of the general formula:

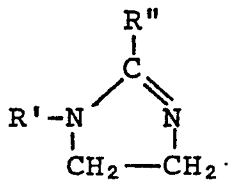

where R' is hydrogen or a hydrocarbon based radical and R" is an aliphatic or alicyclic hydrocarbon based radical.

2. The additive of Claim 1 wherein (A) is an aliphatic hydrocarbon based substituted succinic acylating agent wherein the aliphatic hydrocarbon based substituent is a straight-chain or branched-chain alkyl or alkenyl radical containing from about 4 to about 100 carbon atoms and (B) is an imidazoline of the general formula:

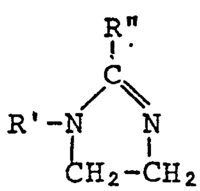

where R' is a straight-chain or branched-chain aliphatic hydrocarbon based radical substituted with -OH or $-NH_2$ groups and containing from 1 to about 6 carbon atoms and R" is an aliphatic or alicyclic hydrocarbon based radical containing from about 10 to about 40 carbon atoms.

3. The additive of Claim 2 wherein reactant (A) is an alkenyl substituted succinic acylating agent where the alkenyl substituent contains from about 8 to about 30 carbon atoms.

4. The additive of Claim 2 or 3 wherein reactant (B) is an imidazoline of the general formula:

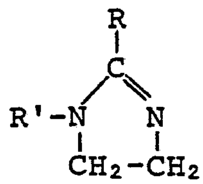

where R' is a straight-chain or branched-chain hydroxyalkyl radical containing from about 1 to about 6 carbon atoms and R" is a straight-chain or branched-chain aliphatic hydrocarbon based radical containing from about 10 to about 20 carbon atoms.

5. The additive of Claim 3 or 4 wherein reactant (A) is an alkenyl substituted succinic anhydride wherein said alkenyl substituent contains from about 9 to about 15 carbon atoms.

6. The additive of Claim 4 or 5 wherein reactant (B) is an imidazoline of the general formula:

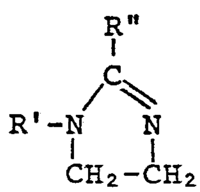

where R' contains from about 2 to about 4 carbon atoms and R" is a straight-chain or branched-chain alkenyl radical containing from about 10 to about 20 carbon atoms.

7. The additive of Claim 5 or 6 wherein reactant (A) is an alkenyl substituted succinic anhydride wherein said alkenyl substituent contains about 12 carbon atoms.

8. The additive of Claim 6 or 7 wherein reactant (B) is an imidazoline of the general formula:

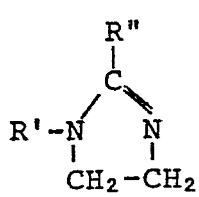

9. A lubricant or fuel composition comprising a major proportion of a lubricant or normally liquid fuel and a minor proportion of at least one additive according to any preceding claim.

10. The fuel composition of Claim 9, in which the normally liquid fuel is gasoline.

11. The fuel composition of Claim 9 in which the normally liquid fuel is methanol, ethanol or hydrated ethanol.

12. The fuel composition of Claim 9 in which the normally liquid fuel is a mixture of gasoline and methanol.

13. The fuel composition of Claim 9 in which the normally liquid fuel is a mixture of gasoline and ethanol.

14. The fuel composition of Claim 9 in which the normally liquid fuel is a mixture of gasoline and hydrated ethanol.

15. A concentrate comprising a normally liquid diluent or fuel and from 20 to 90 percent by weight of an additive according to any of claims 1 to 8.